# EUROPÄISCHE PATENTSCHRIFT

(11) **EP 3 053 646 B1**
(45) Veröffentlichungstag und Bekanntmachung des Hinweises auf die Patenterteilung: **18.04.2018**
(21) Anmeldenummer: 16153662.8
(22) Anmeldetag: 01.02.2016
(51) Int. Cl.: B01J 19/00, B01L 3/00, B01L 7/00, C12M 3/06, C12Q 1/04, C12Q 1/18, C12Q 1/44, C12Q 1/02, C12Q 1/14

(54) **VORRICHTUNG UND VERFAHREN ZUR ERFASSUNG DER RESISTENZ VON BAKTERIEN GEGENÜBER EINEM ZU ANALYSIERENDEN WIRKSTOFF UNTER VERWENDUNG EINES MIKROFLUIDIKCHIPS**
DEVICE AND METHOD FOR DETECTING THE RESISTANCE OF BACTERIA TO AN AGENT TO BE ANALYSED USING A MICROFLUIDIC CHIP
DISPOSITIF ET PROCEDE DE DETECTION DE LA RESISTANCE DE BACTERIES PAR RAPPORT A UN AGENT ACTIF A ANALYSER A L'AIDE D'UNE PUCE MICROFLUIDIQUE

(30) Priorität: 04.02.2015 EP 15153843; 10.02.2015 DE 102015202353
(43) Veröffentlichungstag der Anmeldung: 10.08.2016
(73) Patentinhaber: Fraunhofer-Gesellschaft zur Förderung der angewandten Forschung e.V., 80686 München (DE)
(72) Erfinder: SCHMIDT, Jennifer, 93167 Falkenstein (DE); OHLANDER, Anna, 81379 München (DE)
(74) Vertreter: Stöckeler, Ferdinand

(56) Entgegenhaltungen:
- WO-A1-01/79528
- WO-A2-2009/131686
- DE-B3-102015 202 353
- US-A1- 2005 003 346
- US-A1- 2014 235 490
- ALICIA D. POWERS ET AL: "Multiplexed tyrosine kinase activity detection in cancer cells using a hydrogel immobilized substrate", ANALYTICAL AND BIOANALYTICAL CHEMISTRY, Bd. 405, Nr. 16, 27. April 2013 (2013-04-27), Seiten 5489-5499, XP055282225, DE ISSN: 1618-2642, DOI: 10.1007/s00216-013-6963-5
- MAXIMILIAN FOCKE ET AL: "Lab-on-a-Foil: microfluidics on thin and flexible films", LAB ON A CHIP, Bd. 10, Nr. 11, 1. Januar 2010 (2010-01-01), Seite 1365, XP055046349, ISSN: 1473-0197, DOI: 10.1039/c001195a

## Beschreibung

Die vorliegende Erfindung befasst sich mit Vorrichtungen und Verfahren zur Erfassung, ob Bakterien gegen einen zu analysierenden Wirkstoff resistent sind, unter Verwendung eines Mikrofluidikchips.

Der großzügige Einsatz von Antibiotika beispielsweise im medizinischen Bereich und im Bereich der Tiermast, des Ackerbaus und der Viehzucht, sowie deren unsachgemäße Verwendung haben zur Folge, dass die Bakterien im Laufe der Jahre ausgeprägte Resistenzen gegen die eingesetzten Wirkstoffe entwickelt haben. Infektionskrankheiten sind jährlich für mehr als zehn Millionen Todesfälle verantwortlich, aber die rasante Resistenzentwicklung vieler Krankheitserreger schränkt die Therapiemöglichkeiten immer weiter ein.

Besonders viele Infektionen werden ausgelöst durch resistente Stämme der Species Staphylococcus aureus. Im Zeitraum von 1999 bis 2004 stieg beispielsweise der Anteil an Methicillin-resistenten Staphylokokken (MRSA) auf konstante 20 Prozent. Dieser Problemkeim ist häufig Verursacher nosokomialer Infektionen im Klinikum und ist aber auch oft in Einrichtungen, in denen ältere, immungeschwächte oder immunsupprimierte Personen beherbergt werden, wie beispielsweise im Alten- und Pflegeheim, anzutreffen. Neben Staphylococcus *aureus* existieren inzwischen eine Vielzahl weiterer resistenter Bakterien (wie z.B. Enterokokken, Pneumokokken, Pseudomonas aeruginosa, Campylobacter, EHEC u.a.), deren Infektionskrankheiten nur sehr schwer zu therapieren sind.

Das Testen der Empfindlichkeit von Bakterien auf neue potentielle Wirkstoffe erfolgt derzeit in verschiedenen Verfahren, die eine kulturelle Anzucht der Bakterien erfordern, was jedoch zeit- und kostenintensiv ist, da die Kultivierung unter bestimmten Bedingungen bezüglich Luftfeuchtigkeit, CO₂, Inkubations-Temperatur und -Zeit über mehrere Stunden erfolgen muss.

Zur Bestimmung der in-vitro-Empfindlichkeit von Bakterien auf bestimmte Substanzen gibt es verschiedene Methoden, wie z.B. den Agardiffusionstest, den E-Test und Reihenverdünnungstests, wie die Bouilion- Mikrodilution oder die Agardilution.

### Bouillon-Dilutionstest/Reihenverdünnungstest

Reihenverdünnungstests können unter Verwendung flüssiger Nährmedien (Nährbouillon) in Röhrchen (Makrodilution) und Mikrotiterplatten (Mikrodilution) durchgeführt werden. Daneben werden zur Agardilution auch feste Nährmedien eingesetzt, was jedoch nicht zur Durchführung von Wirkstoffscreenings geeignet ist, weil große Substanzmengen benötigt werden und das Verfahren sehr aufwändig ist.

Die am häufigsten zum Wirkstoffscreening eingesetzte Methode ist die Makrodilutionsmethode. Hier wird in Gegenwart unterschiedlicher Konzentrationen des zu analysierenden Wirkstoffs eine definierte Keimzahl an Bakterien inkubiert. Als minimale Hemmkonzentration (MHK) wird die niedrigste Wirkstoffkonzentration bezeichnet, bei welcher kein sichtbares Wachstum der Bakterien mehr feststellbar ist. Das Wachsum wird anhand der Trübung des Mediums festgestellt. Nachteilig ist, dass vergleichsweise große Wirkstoffmengen eingesetzt werden müssen, die Methode relativ arbeitsaufwändig ist und lange Inkubationszeiten bis zur sichtbaren Trübung des Mediums erforderlich sind. Neben der Makrodilutionsmethode gibt es auch Mikrodilutionsverfahren, welche in der Routinediagnostik zur Erstellung von Antibiogrammen eingesetzt werden. Zur vereinfachten Handhabung sind hierfür bereits Mikrotiterplatten kommerziell erhältlich, Schwarz et al., "Empfindlichkeitsprüfung bakterieller Infektionserreger von Tieren gegenüber antimikrobiellen Wirkstoffen: Methoden zur in-vitro Empfindlichkeitsprüfung und deren Eignung in Hinblick auf die Erarbeitung therapeutisch nutzbarer Ergebnisse"; Berl. Münch. Tierärztl. Wschr. (2003) 116, 353 - 361. Anhand von Dilutionstests sind MBK-Bestimmungen (MBK = minimale bakterizide Konzentration) möglich, diese durchzuführen ist jedoch sehr arbeitsaufwändig.

### Agardiffusionstest

Beim Agardiffusionstest wird der zu analysierende bakterielle Keim homogen in definierter Keimzahl auf ein festes Kulturmedium (i.d.R. Müller-Hinton-Agar) aufgebracht. Der Nährboden wird mit einem mit definierter Wirkstoffmenge bestückten Plättchen belegt. Der Wirkstoff diffundiert aus dem Plättchen in den Nährboden, in dem sich ein Wirkstoffgradient um das Plättchen herum ausbildet. Der zu untersuchende bakterielle Erreger wird in Abhängigkeit von seiner Empfindlichkeit in einem unterschiedlich großen Bereich um das Plättchen herum am Wachstum gehindert. Diesen Bereich bezeichnet man als Hemmhof. Je nach Größe des Hemmhofdurchmessers wird eine qualitative Einteilung der Bakterien in "sensibel", intermediär" oder "resistent" vorgenommen, siehe Schwarz et al.. Diese Methode eignet sich jedoch nicht zur Durchführung von Wirkstoffscreenings im Hochdurchsatz.

### Epsilon-Test (E-Test)

Der Epsilon-Test (E-Test) ist ein Agardiffusionstest, der eine MHK-Wertbestimmung zulässt. Der Wirkstoffträger ist ein Kunststoffstreifen, der einen Wirkstoffgradienten, d.h. eine aufsteigende Antibiotikakonzentration enthält. Genau wie das Plättchen beim Agardiffusionstest wird der Streifen auf eine homogen beimpfte Agarplatte aufgelegt. Nach Ausbildung eines ellipsoiden Hemmhofes bei sensiblen Keimen kann am Schnittpunkt dieses unbewachsenen Bereichs mit dem Teststreifen anhand einer aufgedruckten Skala der MHK-Wert abgelesen werden, wie bei Altreuther et al., "Anmerkungen zum Resistenzmonitoring in der Tiergesundheit"; Berl. Münch. Tierärztl. Wschr. (1997) 110, Seiten 418 - 421 beschrieben ist. Nachteilig ist, dass dieser Test vergleichsweise teuer ist und die veterinärmedizinisch eingesetzten Antibiotika nicht oder nur teilweise angeboten werden. Auch diese Methode ist ungeeignet für das Screening neuer Wirkstoffe.

### Automatisierte Verfahren

Zur Erstellung von Antibiogrammen, d.h. Tests zur Bestimmung der Empfindlichkeit bzw. Resistenz von mikrobiellen Krankheitserregern gegenüber Antibiotika, werden automatisierte Verfahren angewendet, die beispielsweise unter den Bezeichnungen bzw. Marken Mini-API, ATB, VITEK (Bio-Merieux), WalkAway (Dade-Behring) oder Phoenix (Becton Dickinson) bekannt sind. Bei vielen dieser Systeme erfolgt die Auswertung über die Trübung des Mediums nach Wachstum resistenter Bakterien. Hierfür ist ein Inkubationsschritt erforderlich, der häufig mehrere Stunden andauert.

Die Aufgabe der vorliegenden Erfindung besteht darin, Vorrichtungen und Verfahren zu schaffen, die eine Erfassung der Resistenz von Bakterien gegenüber einem zu analysierenden Wirkstoff auf schnelle Art und Weise ermöglichen.

Diese Aufgabe wird durch eine Vorrichtung nach Anspruch 1 und ein Verfahren nach Anspruch 11 gelöst.

Ausführungsbeispiele schaffen eine Vorrichtung gemäß Anspruch 1 zur Erfassung der Resistenz von Bakterien gegenüber einem zu analysierenden Wirkstoff, mit folgenden Merkmalen: einem Mikrofluidikchip, der folgende Merkmale aufweist:
Fluidikstrukturen, die einen Fluideinlass und eine mit dem Fluideinlass fluidisch verbundene Interaktionskammer aufweisen;
ein in der Interaktionskammer immobilisiertes gelabeltes Substrat, das konfiguriert ist, um eine nachweisbare Interaktion mit bakteriellen Faktoren von Bakterien einzugehen, so dass durch Einbringen eines Nährmediums, das einen zu analysierenden Wirkstoff und die Bakterien enthält, in die Interaktionskammer und durch Detektieren des Ergebnisses der nachweisbaren Interaktion feststellbar ist, ob die Bakterien gegen den zu analysierenden Wirkstoff resistent sind,
wobei ein Hohlraum-Volumen der Fluidikstrukturen nicht größer als 100 µl ist;
einem Detektor, der konfiguriert ist, um Informationen zu erfassen, die ein Maß dafür sind, ob die nachweisbare Interaktion stattgefunden hat oder nicht; und
einem Analysator, der ausgebildet ist, um basierend auf den erfassten Informationen eine Anzeige bezüglich der Resistenz der Bakterien gegen den zu analysierenden Wirkstoff auszugeben.

Ausführungsbeispiele schaffen ein Verfahren gemäß Anspruch 11 zur Erfassung der Resistenz von Bakterien gegenüber einem zu analysierenden Wirkstoff, mit folgenden Merkmalen:
Einbringen eines Nährmediums, das einen zu analysierenden Wirkstoff und Bakterien enthält, in die Interaktionskammer eines Mikrofluidikchips, der Fluidikstrukturen, die einen Fluideinlass und eine mit dem Fluideinlass fluidisch verbundene Interaktionskammer aufweisen, und ein in der Interaktionskammer immobilisiertes gelabeltes Substrat aufweist, das konfiguriert ist, um eine nachweisbare Interaktion mit bakteriellen Faktoren von Bakterien einzugehen, wobei ein Hohlraum-Volumen der Fluidikstrukturen nicht größer als 100 µl ist;
Erfassen von Informationen, die ein Maß dafür sind, ob die nachweisbare Interaktion stattgefunden hat oder nicht; und
Ausgeben einer Anzeige, ob resistente Bakterien vorliegen, basierend auf den erfassten Informationen.

Ausführungsbeispiele basieren auf der Erkenntnis, dass es unter Verwendung eines Mikrofluidikchips, der ausgelegt ist, um kleine Flüssigkeitsvolumina in einer Größenordnung von 100 µl und darunter, zu handhaben, und einem in dem Mikrofluidikchip immobilisierten gelabelten Substrat möglich ist, mit gegenüber bekannten Verfahren reduziertem Zeitaufwand sowie einer geringen Probenmenge die Resistenz von Bakterien gegenüber einem zu analysierenden Wirkstoff zu erfassen.

Ausführungsbeispiele der Erfindung werden nachfolgend unter anderem Bezug nehmen auf die beiliegenden Zeichnungen näher erläutert. Es zeigen:
- Fig. 1: eine schematische Darstellung eines Ausführungsbeispiels einer Vorrichtung zur Erfassung der Resistenz von Bakterien gegenüber einem zu analysierenden Wirkstoff;
- Figs. 2 bis 4: schematische Darstellungen von Mikrofluidikchips;
- Fig. 5: eine schematische Darstellung zur Erläuterung eines Ausführungsbeispiels einer Vorrichtung und eines Verfahrens zur Erfassung der Resistenz von Bakterien gegenüber einem zu analysierenden Wirkstoff; und
- Fig. 6: ein Flussdiagramm, das ein Ausführungsbeispiel eines hierin beschriebenen Verfahrens.

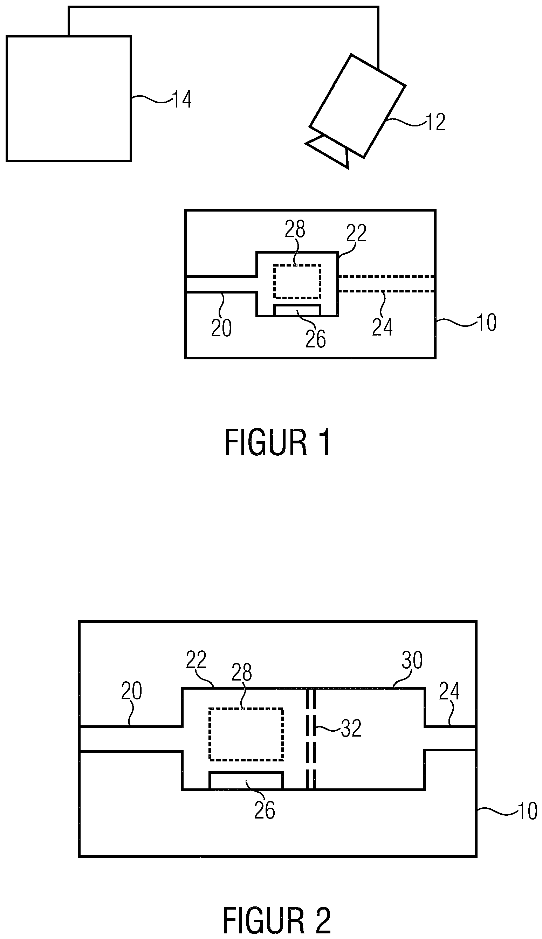

Eine schematische Darstellung einer Vorrichtung zur Erfassung der Resistenz von Bakterien gegenüber einem zu analysierenden Wirkstoff ist in Fig. 1 gezeigt. Die Vorrichtung weist einen mikrofluidischen Chip 10, einen Detektor 12 und einen Analysator 14 auf. Der mikrofluidische Chip 10 ist als schematische Draufsicht dargestellt und weist Fluidikstrukturen auf, die einen Einlass 20 und eine Fluidkammer (Interaktionskammer) 22 aufweisen. Optional weisen die Fluidikstrukturen auch eine Auslass 24 auf, wie durch gestrichelte Linien in Fig. 1 dargestellt ist. In den Figuren ist die Fluidkammer mit einem größeren Querschnitt (in Strömungsrichtung von dem Einlass her) als der Einlass (oder Auslass) dargestellt. Die Fluidkammer 22 kann auch den gleichen Querschnitt aufweisen wie der Einlass(kanal) 20 und der Auslass(kanal) 24. Wie ferner in Fig. 1 durch gestrichelte Linien gezeigt ist, kann ein Heizelement 28 vorgesehen sein, um den Inhalt der Fluidkammer 22 zu heizen. Das Heizelement 28 kann in den Mikrofluidikchip 10 integriert sein oder separat davon vorgesehen sein. Das Heizelement kann vorgesehen sein, um lokal die Fluidkammer 22 und den Inhalt derselben zu heizen oder global den gesamten Mikrofluidikchip 10 zu heizen. Heizelemente, wie sie hierin beschrieben sind, können beispielsweise unter Verwendung von Kupfermaschenheizern implementiert werden, wie sie bei Ohlander et al., "Genotyping of single nucleotide polymorphisms by melting curve analysis using thin film semi-transparent heaters integrated in a lab-on-foil system", Lab Chip (2013), 13, Seiten 2075 - 2082, beschrieben sind.

Das gelabelte Substrat ist mit einem Stoff (Label) gelabelt und interagiert mit bakteriellen Faktoren, wobei diese Interaktion nachweisbar ist. Bei Ausführungsbeispielen kann das Substrat mit Fluoreszenzfarbstoffen (Fluorophoren) gelabelt sein. Bei alternativen Ausführungsbeispielen kann das Substrat mit Absorptionsfarbstoffen oder radioaktiven Stoffen markiert sein. Bei Ausführungsbeispielen werden bei der Interaktion des gelabelten Substrats mit den bakteriellen Faktoren, das Label oder gelabelte Substratfragmente von dem gelabelten Substrat gelöst. Bei Ausführungsbeispielen bewirkt die Interaktion eine Farbänderung des gelabelten Substrats. In jedem Fall wird durch die Interaktion eine Eigenschaft des gelabelten Substrats verändert, die durch den Detektor erfassbar ist, um basierend darauf die Resistenz von Bakterien gegenüber einem zu analysierenden Wirkstoff zu erfassen.

Das gelabelte Substrat bzw. die gelabelten Substrate können neben der festen Anbindung an einer Wand der Fluidkammer auch an faserigen Strukturen, die in die Reaktionskammer eingebracht und immobilisiert werden, bzw. an partikulären Systemen, die in die Fluidkammer eingebracht und immobilisiert werden, angebracht sein. Beispielsweise können die faserigen Strukturen bzw. partikulären Systeme unter Verwendung einer Rückhalteeinrichtung in der Fluidkammer immobilisiert werden. Das bringt den Vorteil einer Oberflächenvergrößerung mit sich und die Substrate sind gegebenfalls sterisch besser zugänglich für die bakteriellen Faktoren.

Ausführungsbeispiele können für ein Wirkstoffscreening zur Identifikation neuer, potentieller Substanzen mit antimikrobieller Wirkung gegen MRSA ausgelegt sein.

Gleichermaßen können auch andere Bakterien-Spezies unter Verwendung des hierin beschriebenen Ansatzes untersucht werden, wobei lediglich das in der interaktionskammer, die auch als Reaktionskammer bezeichnet werden kann, immobilisierte Substrat an die Ausstattung der Bakterien mit unterschiedlichen bakteriellen Faktoren anzupassen sind. Diese bakteriellen Faktoren können beispielsweise Enzyme sein. Wenn sichergestellt ist, dass mit Reinkulturen gearbeitet wird, eignen sich neben Virulenzfaktoren wie DNase, Hyaluoronidase, u.a., auch einfache Stoffwechselenzyme.

Unter einem zu analysierenden Wirkstoff kann dabei hierin eine potenziell antimikrobiell wirkende Substanz verstanden werden. Insbesondere sind darunter auch zu analysierende Substanzen zu verstehen, deren Wirkung bei der Suche nach neuen antimikrobakteriellen Wirkstoffen noch nicht bekannt ist. Ferner sind darunter auch Antibiotika zu verstehen.

Gemäß Ausführungsbeispielen der Erfindung wird somit ein Mikrofluidikchip zur Erfassung der Resistenz von Bakterien gegenüber einem zu analysierenden Wirkstoff eingesetzt. Unter einem Mikrofluidikchip ist dabei ein Chip zu verstehen, der Fluidikstrukturen aufweist, die Abmessungen im Millimeter- oder Sub-Millimeter-Bereich aufweisen können, und somit geeignet sind, sehr kleine Fluidvolumina im µl-Bereich zu handhaben.

Bei Ausführungsbeispielen der Erfindung kann der Mikrofluidikchip ein Folien-basierter Mikrofluidikchip sein, der aus mehreren Lagen von Kunststofffolien aufgebaut ist, die in einer Weise strukturiert sind, dass sich durch Aufeinanderbringen der einzelnen Kunststofffolien (Folienschichten) die Fluidikstrukturen ergeben. Die Fluidikstrukturen weisen einen Einlass und eine Fluidkammer auf. Die Fluidikstrukturen können einen mikrofluidischen Kanal bilden, der einen Einlass und einen Auslass aufweist, wobei ein mittlerer Abschnitt des zu Kanals eine Fluidkammer darstellt. Die Kunststofffolien können beispielsweise mittels Laser strukturiert sein.

Bei alternativen Ausführungsbeispielen kann der Mikrofluidikchip auch in klassischen Spritzguss-Technik implementiert werden. Jedoch bieten die Verwendung von KunststoffFolien und die damit verbundene Möglichkeit Rolle-zu-Rolle-Fabrikationstechniken zu verwenden, an Stelle von Spritzguss-Verfahren grundsätzlich eine Vielzahl von Vorteilen.

Elemente, wie beispielsweise Heizer können integriert werden, wie dies beispielsweise bei Ohlander et al. (2013) beschrieben ist. Auch die Immobilisierung von gelabelten Substraten bzw. einer gelabelten Beschichtung kann leicht vorgenommen werden, wie dies beispielsweise bei Sun et al., "Direct immobilization of DNA probes on non-modified plastics by UV irradiation and integration in microfluidic devices for rapid bioassay", Anal Bioanal Chem (2012), 402, Seiten 741 - 748, beschrieben ist. Die kostengünstige Herstellung dieser Chips in sehr hoher Stückzahl (z.B. im industriellen Maßstab) ist leicht durchführbar. Da für Folien-basierte Chips keine teuren, aufwändigen Formteile gepresst werden müssen, sind sie leicht adaptierbar an neue Bedingungen (oder Nachweisstrategien), was bei der Prototypen-Entwicklung von Vorteil ist, und in Bezug auf die spätere Verwendung sehr flexible Einsatzmöglichkeiten offenbart.

Im Betrieb werden ein Nährmedium, die Bakterien und der zu analysierende Wirkstoff in die Interaktionskammer eingebracht. Bei Ausführungsbeispielen kann eine Suspension, die eine definierte Keimzahl der Bakterien, den zu analysierenden Wirkstoff und das Nährmedium enthält, in die Interaktionskammer eingebracht werden. Das Einbringen kann beispielsweise automatisiert erfolgen, wobei zu diesem Zweck der Einlass mit entsprechenden Chip-externen fluidischen Strukturen (nicht gezeigt) gekoppelt sein kann. Alternativ kann das Einbringen auch manuell erfolgen. In der Interaktionskammer kann die Suspension auf eine Temperatur geheizt werden, bei der die Interaktion zwischen dem gelabelten Substrat und den bakteriellen Faktoren stattfindet, falls die bakteriellen Faktoren vorhanden sind, also falls die Bakterien gegen den zu analysierenden Wirkstoff resistent sind. Falls die Bakterien nicht (sensitiv) oder nur wenig (intermediär) resistent sind, wird die Interaktion nicht oder nur in geringem Maße stattfinden. Der Detektor ist ausgelegt, um Informationen zu erfassen, die ein Maß dafür sind, ob und vorzugsweise in welchem Umfang die Interaktion stattgefunden hat. Der Detektor liefert diese Informationen zu dem Analysator, der ausgelegt sein kann, um die Informationen mit einem oder mehreren Schwellenwerten zu vergleichen und abhängig von dem Vergleich eine Anzeige auszugeben, ob die Bakterien gegen den zu analysierenden Wirkstoff resistent sind oder nicht. Bei Ausführungsbeispielen kann der Analysator die Informationen mit einem Schwellwert vergleichen und abhängig von dem Vergleich eine Anzeige ausgeben, dass die Bakterien gegen den zu analysierenden Wirkstoff resistent sind oder nicht. Bei Ausführungsbeispielen kann der Analysator ausgelegt sein, um die Informationen mit mehreren Schwellwerten zu vergleichen, um eine quantitative Aussage hinsichtlich der Resistenz zu ermöglichen, beispielsweise ob die Bakterien gegen den zu analysierenden Wirkstoff resistent, intermediär oder sensibel sind. Beispielsweise werden die Bakterien als resistent bewertet, wenn der Vergleich ergibt, dass ein erster Schwellwert nicht überschritten wird, werden als intermediär bewertet, wenn ein zweiter Schwellwert, der höher als der erste Schwellwert ist, nicht überschritten wird, und werden als sensibel bewertet, wenn der zweite Schwellwert überschritten wird. Bei Ausführungsbeispielen kann auch eine höhere Anzahl von Schwellwerten verwendet werden.

Der Detektor ist ausgelegt, um die jeweilige Interaktion zu erfassen. Der Detektor ist dabei an die Art des gelabelten Substrats und die Art der Interaktion angepasst. Werden Fluorophore oder Absorbtionsfarbstoffe als Label verwendet, kann der Detektor einen Fotomultiplizierer oder einen Ein-Photon-Detektor aufweisen. Solche Detektoren sind gegenüber einem Fluoreszenzsignal hochempfindlich und können in der Lage sein, sogar einzelne Photonen nachzuweisen, was eine Verkürzung der Nachweiszeit ermöglichen kann. Insbesondere ist eine mehrstündige Kultivierung zur Vervielfachung der Probenmengen nicht nötig. Bei Ausführungsbeispielen kann ein Silizium-Photomultiplizierer (SiPM) zur Detektion verwendet werden. Siliziumbauelemente sind interessant, weil sie sich miniaturisieren lassen, wenig Energie verbrauchen und zugleich kostengünstig sind. Bei alternativen Ausführungsbeispielen könne hochauflösende Kameras als Detektor verwendet werden. Abhängig von der Art des verwendeten gelabelten Substrats können bei wiederum alternativen Ausführungsbeispielen Detektoren verwendet werden, die zur Erfassung von radioaktiver Strahlung geeignet sind.

Der Analysator kann durch eine Recheneinrichtung, beispielsweise eine Computer, implementiert sein, die konfiguriert bzw. programmiert ist, um eine entsprechende Analyse der von dem Detektor kommenden Informationen durchzuführen. Bei Ausführungsbeispielen kann der Analysator einen Prozessor, beispielsweise eine Mikroprozessor, aufweisen, der über einen Kommunikationsbus mit einem Speicher gekoppelt ist, wobei der Speicher maschinenlesbare Befehle speichert, die durch den Prozessor ausgeführt werden können, um die hierin beschriebene Funktionalität zu liefern. Der Analysator kann ferner eine Anzeigevorrichtung aufweisen, auf der das Ergebnis der Untersuchung angezeigt werden kann. Bei der Anzeigevorrichtung kann es sich beispielsweise um einen Bildschirm oder einen Drucker handeln.

Bei dem in Fig. 1 gezeigten Ausführungsbeispiel kann der Detektor 12 angeordnet sein, um eine in der Interaktionskammer 22 auftretende Fluoreszenz zu erfassen. Das gelabelte Substrat kann bei dabei mit Fluorophoren und Quenchern gelabelt sein, so dass eine Fluoreszenz erst nach einer enzymatischen Spaltung, die durch in der Interaktionskammer 22 befindliche Bakterien, die gegen einen zu analysierenden Wirkstoff resistent sind, bzw. deren bakteriellen Faktoren bewirkt wird, auftrifft. Somit kann eine Anreicherung, Reaktion (Interaktion) und Signal-Detektion in einer einzigen Kammer gleichzeitig erfolgen. Ein solcher Aufbau ermöglicht eine einfache Durchführung dynamischer Messungen.

Ein Ausführungsbeispiel, bei dem Streulichteffekte durch Partikel in der Suspension verhindert werden können, wird nun Bezug nehmend auf Fig. 2 erläutert, die einen Mikrofluidikchip 10 für ein solches Ausführungsbeispiel zeigt. Die Fluidikstrukturen des in Fig. 2 gezeigten Mikrofluidikchips 10 weisen den Einlass 20, die Interaktionskammer 22, eine Detektionskammer 30, die mit der Interaktionskammer 22 fluidisch über eine Rückhalteeinrichtung 32 gekoppelt ist, und den Auslass 24 auf. Die Rückhalteeinrichtung ist ausgelegt, um Label und/oder Substratfragmente, die aufgrund der Interaktion von dem gelabelten Substrat gelöst werden, passieren zu lassen und die Bakterien zurückzuhalten. Die Rückhalteeinrichtung kann beispielsweise durch ein Filter mit einer entsprechenden Porengröße (von beispielsweise 0,2 µm) implementiert sein. Bei diesem Ausführungsbeispiel können die Label des gelabelten Substrats beispielsweise durch Fluorophore gebildet sein, die durch die Interaktion von dem Substrat gelöst werden.

Im Betrieb kann wiederum eine Suspension in die Interaktionskammer 22 eingebracht und geheizt werden, wie oben beschrieben wurde. Nachfolgend kann eine Strömung durch die Fluidikstrukturen bewirkt werden, indem beispielsweise über den Einlass 20 ein Druck durch eine Chip-externe Pumpe bzw. einen Chip-externen Druckgeber angelegt wird. Alternativ kann eine Pumpe oder ein Druckgeber auf dem Chip integriert sein. Dadurch werden gelöste Label bzw. gelöste gelabelte Substratfragmente durch die Rückhalteeinrichtung 32 getrieben, während Bakterien in der Rückhalteeinrichtung 32 zurückgehalten werden. Dadurch können die Stoffe bzw. Label, die die Rückhalteeinrichtung 32 passiert haben, in der Detektionskammer 30 unter Verwendung des Detektors (in Fig. 2 nicht gezeigt), der auf die Detektionskammer 30 ausgerichtet ist, erfasst werden, ohne dass Streulichteffekte durch die Bakterien oder andere Partikel, die die Rückhalteeinrichtung 32 nicht passieren können, bewirkt werden.

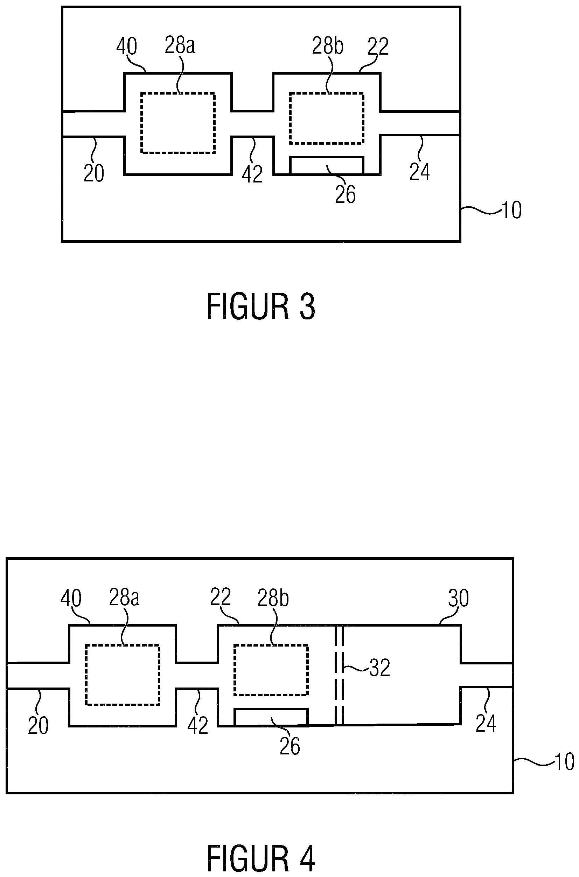

Fig. 3 zeigt einen mikrofluidischen Chip 10 für ein alternatives Ausführungsbeispiel, bei dem die Fluidikstrukturen eine Anreicherungskammer 40 aufweisen, die fluidisch zwischen den Einlass 20 und die Interaktionskammer 22 geschaltet ist. Die Anreicherungskammer 40 ist über Fluidkanal 42 mit der Interaktionskammer 22 verbunden. Ein erstes Heizelement 28a, das ausgelegt ist, um den Inhalt der Anreicherungskammer zu heizen, und ein zweites Heizelement 28b, das ausgelegt ist, um den Inhalt der Interaktionskammer 22 zu heizen, sind vorgesehen. Die Heizelemente 28a und 28b können jeweils in den Chip integriert oder Chip-extern vorgesehen sein. Das Heizelement 28a ist ausgelegt, um den Inhalt der Anreicherungskammer auf eine für eine Anreicherung der Bakterien in dem Nährmedium geeignete Temperatur zu heizen, und das Heizelement 28b ist ausgelegt, um den Inhalt der Interaktionskammer auf eine für die Interaktion geeignete Temperatur zu heizen.

Im Betrieb kann eine Suspension zunächst in die Anreicherungskammer 40 eingebracht werden, wo sie auf eine Anreicherungstemperatur, die für eine Kultivierung der Bakterien geeignet ist, z.B. 37°C, erwärmt wird. Nach der Kultivierung wird die Suspension in die Interaktionskammer 22 gepumpt, beispielsweise durch eine Chip-interne oder Chip-externe Pumpeinrichtung. Dort kann die kultivierte Suspension auf eine für die Interaktion, beispielsweise eine enzymatische Aktivität geeignete Temperatur, wie z.B. 45°C, erwärmt werden. Somit ist es möglich, die Temperatur für den jeweiligen Vorgang zu optimieren. Die Erfassung der Interaktion kann dann auf die oben Bezug nehmend auf Fig. 1 beschriebene Weise erfolgen.

Fig. 4 zeigt einen Mikrofluidikchip, dessen Fluidikstrukturen eine Kombination der Bezug nehmend auf die Figuren 2 und 3 beschriebenen Merkmale aufweisen. Die Erfassung von Informationen, die ein Maß dafür sind, ob die nachweisbare Interaktion sattgefunden hat oder nicht, kann dabei auf die Bezug nehmend auf Fig. 2 beschriebene Weise erfolgen.

Bei alternativen Ausführungsbeispielen kann der Mikrofluidikchip mehrere mit unterschiedlichen Labeln gelabelte Substrate aufweisen, die in einer oder mehreren Fluidkammern des Mikrofluidikchips immobilisiert sind. Es ist somit möglich, auf einem Chip mehrere unterschiedliche Nachweisreaktionen parallel durchzuführen. Um am Detektor zwischen den unterschiedlichen Nachweisreaktionen diskriminieren zu können, könne hierfür beispielsweise unterschiedliche Fluoreszenz-Label (mit unterschiedlichen spektralen Eigenschaften) verwendet werden. Beispielsweise kann ein erster bakterieller Faktor A einen Fluorophor A freisetzen, während ein zweiter bakterieller Faktor B einen Fluorophor B freisetzen kann, usw.

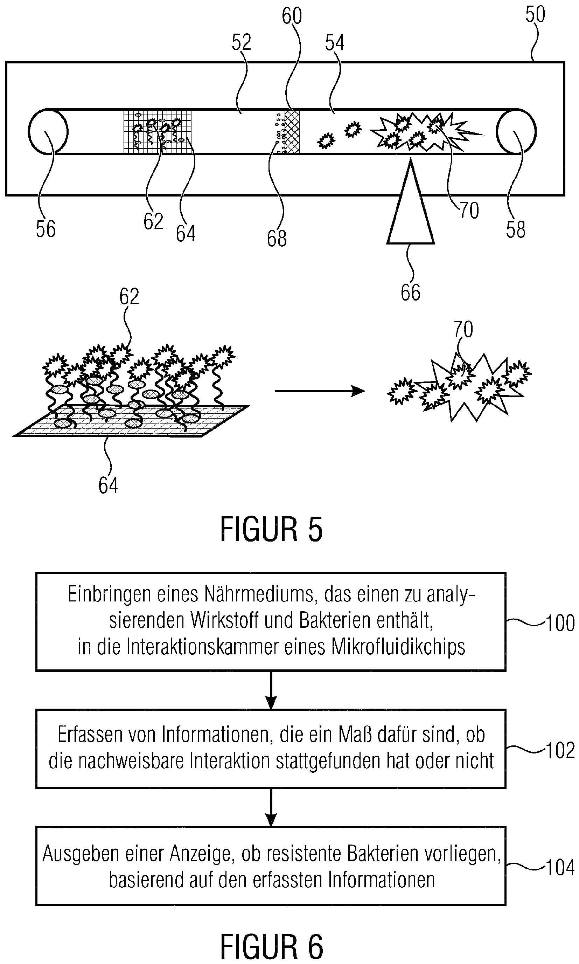

Im folgenden wird ein Ausführungsbeispiel unter Verwendung eines Folien-basierten Chips exemplarisch am Beispiel Wirkstoffscreening zur Identifikation neuer, potenzieller Substanzen mit antimikrobakterieller Wirkung gegen MRSA Bezug nehmend auf Fig. 5 beschrieben. Der Chip 50 weist Fluidikstrukturen auf, die zwei Kammern aufweisen, eine erste Kammer 52, die als Anreicherungs- und Reaktions-Kammer (Interaktionskammer) dient, und eine zweite Kammer, die als Detektionskammer 54 dient. Ferner weisen die Fluidikstrukturen einen Einlass 56 und einen Auslass 58 auf. Die Kammern 52 und 54 sind über einen mikrofluidischen Kanal miteinander verbunden, wobei Kammern und Kanal einen identischen Strömungsquerschnitt aufweisen. Zwischen den Kammern 52 und 54 befindet sich eine Vorrichtung 60, beispielsweise eine Filtermembran, die dem Zurückhalten von Partikeln und Bakterien aus einem Probenstrom zwischen den Kammern 52 und 54 dient. Im Bereich der Anreicherungs- und Reaktionskammer 52 befindet sich auf der Folie immobilisierte DNA 62 (beispielsweise gespottete pTpC-Oligonukleotide), die mit einem Fluoreszenz-Label markiert ist, siehe z.B. Sun et al., 2012. Auf der Rückseite der Folie, auf deren Vorderseite die Kammer 52 gebildet ist, befindet sich die Struktur 64 eines Heizelements, bei dem es sich beispielsweise um einen gedruckten Kupfermaschenheizer, welcher über Leiterbahnen angesteuert und entsprechend temperiert werden kann, handeln kann, wie er bei Ohlander et al., 2013, beschrieben ist.

Insofern kann der Chip 50 einen Aufbau aufweisen, wie er oben Bezug nehmend auf Fig. 2 beschrieben wurde.

Soll die Empfindlichkeit von MRSA-Stämmen gegenüber neuen, potentiell antimikrobiellen Wirkstoffen analysiert werden, wird eine definierte Keimzahl MRSA-Bakterien mit dem zu testenden Wirkstoff in definierter Konzentration, z.B. als eine Konzentrationsreihe zur Bestimmung von MHK/MBK, in einem kleinen Volumen Nährmedium zusammengebracht. Das Gesamtvolumen des Chips (also das Volumen, das zur Befüllung mit Fluid bzw. Flüssigkeit zur Verfügung steht, das hierin auch als Hohlraumvolumen bezeichnet wird, beträgt nicht mehr als 100 µL und kann bei Ausführungsbeispielen in einem Bereich von 10 - 50µL liegen. Die Fluidikstrukturen des Chips werden im Folgenden mit dieser Suspension beladen, wobei dieser Schritt automatisiert erfolgen kann. In der Anreicherungs- und Reaktionskammer wird durch das Heizelement eine für Kulturbedingungen optimale Temperatur eingestellt, beispielsweise 37°C, sodass die Bakterien, die gegen den zu analysierenden Wirkstoff resistent sind, sich vermehren und entsprechende bakterielle Faktoren bilden können. MRSA-Stämme z.B. exprimieren den Virulenzfaktor DNase, ein Enzym, welches die Spaltung von DNA-Strängen enzymatisch katalysiert. Dieses Enzym wird von den Bakterien an das sie umgebende Medium abgegeben. Zeitgleich zur exponentiellen Vermehrung der Bakterien wird das Fluoreszenz-gelabelte Substrat enzymatisch in der Anreicherungs-und Reaktionskammer gespalten und somit der Fluorophor freigesetzt. Zur Erlangung maximaler enzymatischer Aktivität kann die Inkubationstemperatur beispielsweise 10 Minuten vor dem Weitertransport der Bulk-Phase in die Detektionskammer 54 auf 39°C bzw. auf 45°C erhöht werden, je nach Temperaturoptimum des Enzyms. Hierdurch kann eine weitere Beschleunigung des Tests erlangt werden.

Nach einer definierten Zeit, abhängig von der verwendeten Keimzahl und Spezies, wird der Inhalt der Kammer 52 weitergepumpt in die Detektionskammer 54, in der die gelösten Label 70, d.h. die Fluoreszenz, hochempfindlich gemessen und quantifiziert werden kann, beispielsweise mittels eines Fotomultiplizierers 66. Die Rückhaltevorrichtung 60 zwischen den Kammern 52 und 54 stellt sicher, dass keine partikulären Strukturen oder Bakterien 68, die Streulichtsignale erzeugen können, in die Detektionskammer 54 gelangen.

Ist keine Fluoreszenz detektierbar, so hat der Test-Wirkstoff antibakterielle Wirkung, wobei entsprechende Kontrollen mitgeführt werden können. Die Inkubationszeit, die herkömmliche Verfahren zum Wirkstoffscreening benötigen, wird mit Hilfe dieses Folien-basierten Chips weit unterschritten, denn das Fluoreszenzsignal kann extrem sensitiv und somit sehr viel früher detektiert werden als - wie bislang üblich - eine durch Bakterienzellen verursachte Trübung des Mediums.

Alternativ kann statt der Zunahme der Fluoreszenzintensität in der Detektionskammer auch die Signalabnahme in der Anreicherungs- und Reaktionskammer vermessen werden.

Neben einer solchen Empfindlichkeitsprüfung können bei Ausführungsbeispielen solche Chips auch eingesetzt werden zum Nachweis resistenter Bakterien beispielsweise in Lebensmitteln. Hier ist gegebenenfalls nach Anreicherung der Bakterien, z.B. mittels partikulärer Systeme oder Filtration eine Inkubation des zu untersuchenden Materials (z.B. Lebensmittelproben, Waschwasser von Schlachtungen oder von Kräutern und Gemüse) in einem Medium, dem bestimmte Antibiotika (abhängig davon, welche resistenten Bakterien nachgewiesen werden sollen) zugesetzt sind, erforderlich. Resistente Bakterien überleben und können auf dem Chip nachgewiesen.

Fig. 6 stellt schematisch ein Verfahren zur Erfassung der Resistenz von Bakterien gegenüber einem zu analysierenden Wirkstoff gemäß einem Ausführungsbeispiel dar.

In einem Schritt 100 wird Nährmedium, das einen zu analysierenden Wirkstoff und Bakterien enthält, in die Interaktionskammer eines Mikrofluidikchips eingebracht, der Fluidikstrukturen, die einen Fluideinlass und eine mit dem Fluideinlass fluidisch verbundene Interaktionskammer aufweisen, und ein in der Interaktionskammer immobilisiertes gelabeltes Substrat aufweist, das konfiguriert ist, um eine nachweisbare Interaktion mit bakteriellen Faktoren von Bakterien einzugehen, wobei ein Hohlraum-Volumen der Fluidikstrukturen nicht größer als 100 µl ist. Dabei kann ein definiertes Volumen Nährmedium eine definierte Keimzahl von Bakterien und einen definierten Wirkstoff in definierter Konzentration enthalten. Nacheinander oder parallel können Suspensionen mit entsprechendem Nährmedienvolumen und entsprechender Keimzahl von Bakterien und unterschiedlichen Wirkstoffkonzentrationen verwendet werden, beispielsweise um eine Konzentrationsreiche zur MHK/MBK-Bestimmung zu implementieren. In einem Schritt 102 werden Informationen, die ein Maß dafür sind, ob die nachweisbare Interaktion stattgefunden hat oder nicht, erfasst. In einem Schritt 104 wird eine Anzeige, ob resistente Bakterien vorliegen, basierend auf den erfassten Informationen ausgegeben. Diese Anzeige kann beispielsweise angeben, ob die Bakterien bezüglich des zu analysierenden Wirkstoffs resistent, intermediär oder sensitiv sind. Die Anzeige kann auch MHK und MBK angeben.

Mikrofluidikchips, die Folien-basiert sein können, zum Screening antimikrobieller Wirkstoffe (z.B. gegen MRSA) können im Vergleich zu derzeit verwendeten Methoden (i.d.R. Dilutionstests) in deutlich kürzerer Zeit ein Ergebnis liefern. Durch den Einsatz eines extrem empfindlichen (und dennoch kostengünstigen) SiPMs, der in der Lage ist, einzelne Photonen zu erfassen, kann das Fluoreszenzsignal sehr viel eher zu detektieren sein als die herkömmlich gemessene Trübung des Mediums. Das Nachweissystem ist automatisierbar (geeignet für HTS) und miniaturisiert, d.h. es werden nur sehr geringe Wirkstoffmengen zur Empfindlichkeitsprüfung benötigt. Der Chip kann Folien-basiert sein und daher in sehr hoher Stückzahl sehr kostengünstig als Einwegprodukt herstellbar sind. Aufwändige Reinigungsschritte können entfallen. Bei dem oben beschriebenen Ausführungsbeispiel mit Rückhaltevorrichtung im Kanal können Partikel und Bakterienzellen zurückgehalten werden, sodass keine Streulichteffekte bei der Erfassung, beispielsweise der Fluoreszenzmessung, auftreten können. Je nach Ausführungsbeispiel ermöglicht die Integration von zwei oder mehreren unterschiedlich temperierbaren Kupfermaschenheizern (Cu-mesh-heatern) eine Inkubation der Bakterien in Gegenwart von der zu analysierenden Substanz bei optimalen Kulturbedingungen (z.B. 37°C), während die enzymatische Reaktion bei einer anderen optimalen Temperatur (z.B. 45°C) ablaufen kann. Alternativ können auch zwei verschiedene Temperaturen nacheinander in der Anreicherungs- und Reaktionskammer gefahren werden. Die daraus resultierende Erhöhung der Reaktionsgeschwindigkeit ermöglicht weiterhin eine Verkürzung der Messzeit. Da mit diesem Verfahren lebende Bakterien nachgewiesen werden, eignet sich der Chip auch zu Dekontaminationskontrolle.

Ausführungsbeispiele ermöglichen somit den Einsatz von Mikrofluidikchips auf der Suche nach neuartigen, antimikrobiellen Wirkstoffen, mit denen sich beispielsweise Infektionen behandeln lassen, die durch multiresistente Bakterien wie MRSA verursacht werden. Neben MRSA sind auch Empfindlichkeitsprüfungen anderer Bakterien eingeschlossen, wobei auf dem Chip eine passende Nachweisreaktion implementiert sein kann. Neben der Anwendung in der Pharmaindustrie ist auch eine Verwendung bei der Kontrolle von Lebensmitteln denkbar. Beispielsweise Waschwasser aus Schlachtereien oder von Gemüse/Salat/Kräutern kann mit diesem System auf das Vorhandensein resistenter Bakterien untersucht werden. Nach Anreicherung der Bakterien in einem geeigneten Filter bzw. auf einem Partikel-basierten System erfolgt eine Inkubation in Gegenwart eines Antibiotika-enthaltenden Nährmediums und - genau wie beim Wirkstoffscreening - der Nachweis lebender Bakterien im dem Mikrofluidikchip.

Ausführungsbeispiele beziehen sich somit auf einen Folien-basierten Mikrofluidikchip zur Detektion lebender Bakterien, z.B. für die Empfindlichkeitsbestimmung bakterieller Keime. Ausführungsbeispiele beziehen sich somit auf den Einsatz eines Folien-basierten Mikrofluidikchips zum Wirkstoffscreening (z.B. in der Pharmaindustrie) und ermöglichen kostengünstig und schneller als herkömmliche Methoden, die in der Regel auf Trübungsmessungen basieren, die Identifikation neuer potentieller Wirkstoffe zur Behandlung von Infektionen, die durch resistente Bakterien ausgelöst werden. Dabei kann zur Empfindlichkeitsprüfung von Bakterien gegen einen neuen potentiellen Wirkstoff, jeweils eine definierte Keimzahl ausgewählter Bakterien zusammen mit unterschiedlichen Konzentrationen eines neuen potentiellen Wirkstoffs, dessen antimikrobielle Eigenschaften analysiert werden sollen, (z.B. als Konzentrationsreihe) in ein Nährmediumvolumen eingebracht und wie beschrieben untersucht werden, um z.B. MHK und MBK zu bestimmen.

Obwohl manche Aspekte im Zusammenhang mit einer Vorrichtung bzw. der Funktionalität einer Vorrichtung beschrieben wurden, versteht es sich, dass diese Aspekte auch Merkmale eines entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung einer entsprechenden Funktionalität bzw. eines entsprechenden Blocks oder Details oder Merkmals einer entsprechenden Vorrichtung dar.

## Patentansprüche

1. Vorrichtung zur Erfassung der Resistenz von Bakterien gegenüber einem zu analysierenden Wirkstoff, mit folgenden Merkmalen:
einem Mikrofluidikchip (10, 50), der folgende Merkmale aufweist:
Fluidikstrukturen, die einen Fluideinlass (20, 56) und eine mit dem Fluideinlass (20, 56) fluidisch verbundene Interaktionskammer (22, 52) aufweisen;
ein in der Interaktionskammer (22, 52) immobilisiertes gelabeltes Substrat (26, 62), das konfiguriert ist, um eine nachweisbare Interaktion mit bakteriellen Faktoren von Bakterien einzugehen, so dass durch Einbringen eines Nährmediums, das einen zu analysierenden Wirkstoff und die Bakterien enthält, in die Interaktionskammer (22, 52) und durch Detektieren des Ergebnisses der nachweisbaren Interaktion feststellbar ist, ob die Bakterien gegen den zu analysierenden Wirkstoff resistent sind, wobei Bakterien, die gegen den zu analysierenden Wirkstoff resistent sind, entsprechende bakterielle Faktoren bilden können,
wobei ein Hohlraum-Volumen der Fluidikstrukturen nicht größer als 100 µl ist;
einem Detektor (12, 66), der konfiguriert ist, um Informationen zu erfassen, die ein Maß dafür sind, ob die nachweisbare Interaktion stattgefunden hat oder nicht; und
einem Analysator (14), der ausgebildet ist, um basierend auf den erfassten Informationen eine Anzeige bezüglich der Resistenz der Bakterien gegen den zu analysierenden Wirkstoff auszugeben,
wobei das Substrat (26, 62) mit einem Stoff gelabelt ist, der durch die Interaktion mit den bakteriellen Faktoren von dem Substrat (26, 62) gelöst wird, und
wobei die Fluidikstrukturen des Mikrofluidikmoduls eine Detektionskammer (30, 54) aufweisen, die fluidisch mit der Interaktionskammer (22, 52) gekoppelt ist,
wobei eine Rückhaltevorrichtung (32, 60) fluidisch zwischen die Interaktionskammer (22, 52) und die Detektionskammer (30, 54) geschaltet ist, wobei die Rückhaltevorrichtung konfiguriert ist, um Label (70) und/oder gelabelte Substratfragmente, die aufgrund der Interaktion von dem gelabelten Substrat (26, 62) gelöst werden, passieren zu lassen und die Bakterien (68) zurückzuhalten.

2. Vorrichtung nach Anspruch 1, bei der der Analysator (14) ausgebildet ist, um die erfassten Informationen mit einem zumindest einem Schwellwert zu vergleichen und um abhängig von dem Vergleich eine Anzeige auszugeben, ob die Bakterien gegen den zu analysierenden Wirkstoff resistent sind oder nicht, oder bei der der Analysator (14) ausgebildet ist, um die erfassten Informationen mit mehreren Schwellwerten zu vergleichen und um abhängig von dem Vergleich eine Anzeige auszugeben, ob die Bakterien gegen den zu analysierenden Wirkstoff resistent, intermediär oder sensibel sind.

3. Vorrichtung nach Anspruch 1 oder 2, bei der der Mikrofluidikchip (10, 50) eine Mehrzahl von Kunststofffolien aufweist, von denen zumindest eine strukturiert ist, um die Fluidikstrukturen zu definieren.

4. Vorrichtung nach einem der Ansprüche 1 bis 3, bei der der Mikrofluidikchip (10) ferner eine Anreicherungskammer (40) aufweist, die fluidisch zwischen den Fluideinlass (20) und die Interaktionskammer (22) geschaltet ist.

5. Vorrichtung nach Anspruch 4, die ein Heizelement (28a) aufweist, das konfiguriert ist, um einen Inhalt der Anreicherungskammer (40) auf eine für eine Anreicherung der Bakterien in dem Nährmedium geeignete Temperatur zu heizen.

6. Vorrichtung nach einem der Ansprüche 1 bis 5, die ein Heizelement (28, 28b) aufweist, das konfiguriert ist, um einen Inhalt der Interaktionskammer (22, 52) auf eine für die nachweisbare Interaktion geeignete Temperatur zu heizen.

7. Vorrichtung nach einem der Ansprüche 1 bis 6, bei der das Substrat (26, 62) mit Fluoreszenzfarbstoffen, Absorbtionsfarbstoffen oder radioaktiven markierten Labeln gelabelt ist.

8. Vorrichtung nach einem der Ansprüche 1 bis 7, bei der das gelabelte Substrat (26, 62) an einer Wand der Interaktionskammer (22, 52) oder an in der Interaktionskammer (22, 52) immobilisierten faserigen oder partikulären Strukturen angebracht ist.

9. Vorrichtung nach einem der Ansprüche 1 bis 8, bei der der Mikrofluidikchip (10, 50) mehrere mit unterschiedlichen Labeln gelabelte Substrate (26, 62) aufweist, die in einer oder mehreren Fluidkammern des Mikrofluidikchips (10, 50) immobilisiert sind.

10. Vorrichtung nach Anspruch 9, bei der das gelabelte Substrat (26, 62) mit Fluorophoren oder Absorbtionsfarbstoffen gelabelt ist, und wobei der Detektor (12, 66) einen Fotomultiplizierer oder einen Ein-Photon-Detektor aufweist.

11. Verfahren zur Erfassung der Resistenz von Bakterien gegenüber einem zu analysierenden Wirkstoff unter Verwendung einer Vorrichtung nach einem der Ansprüche 1 bis 10, mit folgenden Merkmalen:
Einbringen (100) des Nährmediums, das den zu analysierenden Wirkstoff und Bakterien enthält, in die Interaktionskammer (22, 52) des Mikrofluidikchips (10, 50);
Erfassen (102) von Informationen, die ein Maß dafür sind, ob die nachweisbare Interaktion stattgefunden hat oder nicht; und
Ausgeben (104) einer Anzeige bezüglich der Resistenz der Bakterien gegen den zu analysierenden Wirkstoff basierend auf den erfassten Informationen.

12. Verfahren nach Anspruch 11, das ein Vergleichen der erfassten Informationen mit zumindest einem Schwellwert und das Ausgeben der Anzeige, ob resistente Bakterien vorliegen, basierend auf dem Vergleich aufweist.

13. Verfahren nach Anspruch 12, das ein Vergleichen der erfassten Informationen mit mehreren Schwellwerten und das Ausgeben der Anzeige, ob die Bakterien gegen den zu analysierenden Wirkstoff resistent, intermediär oder sensibel sind, basierend auf dem Vergleich aufweist.

14. Verfahren nach einem der Ansprüche 12 bis 13, wobei die Bakterien bekannte Bakterien sind und wobei das Verfahren ausgelegt ist, um ein Wirkstoffscreening durchzuführen.

## Claims

1. Device for detecting the resistance of bacteria to an active substance to be analyzed, comprising:
a microfluidic chip (10, 50) comprising:
fluidic structures having a fluid inlet (20, 56) and an interaction chamber (22, 52) fluidically connected to the fluid inlet (20, 56);
a labeled substrate (26, 62) that is immobilized inside the interaction chamber (22, 52) and is configured to enter into a verifiable interaction with bacterial factors of bacteria, so that by introducing a culture medium containing an active substance to be analyzed and the bacteria into the interaction chamber (22, 52) and by detecting the result of the verifiable interaction, it can be ascertained whether the bacteria are resistant to the active substance to be analyzed, wherein bacteria that are resistant to the active substance to be analyzed can form respective bacterial factors,
a void volume of the fluidic structures not exceeding 100 µl;
a detector (12, 66) configured to detect information that is a measure of whether or not the verifiable interaction has taken place; and
an analyzer (14) configured to output, on the basis of the detected information, a display with regard to the resistance of the bacteria to the active substance to be analyzed,
wherein the substrate (26, 62) is labeled with a substance that is detached from the substrate (26, 62) by the interaction with the bacterial factors, and
wherein the fluidic structures of the microfluidic module comprise a detection chamber (30, 54) fluidically coupled to the interaction chamber (22, 52), a retention device (32, 60) being fluidically connected between the interaction chamber (22, 52) and the detection chamber (30, 54), the retention device being configured to allow labels (70) and/or labeled substrate fragments, which are detached from the labeled substrate (26, 62) on account of the interaction, to pass through and to retain the bacteria (68).

2. Device as claimed in claim 1, wherein the analyzer (14) is configured to compare the detected information to at least one threshold value and to output a display, as a function of the comparison, as to whether or not the bacteria are resistant to the active substance to be analyzed, or wherein the analyzer (14) is configured to compare the detected information to several threshold values and to output a display, as a function of the comparison, as to whether the bacteria are resistant, intermediate, or sensitive to the active substance to be analyzed.

3. Device as claimed in claim 1 or 2, wherein the microfluidic chip (10, 50) comprises a plurality of plastic foils, at least one of which is structured to define the fluidic structures.

4. Device as claimed in any of claims 1 to 3, wherein the microfluidic chip (10) further comprises an enrichment chamber (40) fluidically connected between the fluid inlet (20) and the interaction chamber (22).

5. Device as claimed in claim 4, comprising a heating element (28a) configured to heat contents of the enrichment chamber (40) to a temperature suited for enriching the bacteria within the culture medium.

6. Device as claimed in any of claims 1 to 5, comprising a heating element (28a, 28b) configured to heat contents of the interaction chamber (22, 52) to a temperature suited for the verifiable interaction.

7. Device as claimed in any of claims 1 to 6, wherein the substrate (26, 62) is labeled with fluorescent dyes, absorption dyes or radioactive marked labels.

8. Device as claimed in any of claims 1 to 7, wherein the labeled substrate (26, 62) is mounted on a wall of the interaction chamber (22, 52) or on fibrous or particulate structures immobilized inside the interaction chamber (22, 52).

9. Device as claimed in any of claims 1 to 8, wherein the microfluidic chip (10, 50) comprises several substrates (26, 62) labeled with different labels and immobilized inside one or more fluid chambers of the microfluidic chip (10, 50).

10. Device as claimed in claim 9, wherein the labeled substrate (26, 62) is labeled with fluorophores or absorption dyes, and wherein the detector (12, 66) comprises a photomultiplier or a one-photon detector.

11. Method of detecting the resistance of bacteria to an active substance to be analyzed using an apparatus as claimed in any of claims 1 to 10, comprising:
introducing (100) the culture medium containing the active substance to be analyzed and bacteria into the interaction chamber (22, 52) of the microfluidic chip (10, 50);
detecting (102) information that is a measure of whether or not the verifiable interaction has taken place; and
outputting (104) a display, on the basis of the detected information, with regard to the resistance of the bacteria to the active substance to be analyzed.

12. Method as claimed in claim 11, comprising comparing the detected information to at least one threshold value and outputting the display, on the basis of the comparison, as to whether there are resistant bacteria.

13. Method as claimed in claim 12, comprising comparing the detected information to several threshold values and outputting the display, on the basis of the comparison, as to whether the bacteria are resistant, intermediate, or sensitive to the active substance to be analyzed.

14. Method as claimed in one of claims 12 to 13, the bacteria being known bacteria and the method being configured to perform an active-substance screening.

## Revendications

1. Dispositif de détention de la résistance de bactéries à un agent actif à analyser, aux caractéristiques suivantes:
une puce microfluidique (10, 50) qui présente les caractéristiques suivantes:
des structures fluidiques qui présentent une entrée de fluide (20, 56) et une chambre d'interaction (22, 52) connectée en fluide à l'entrée de fluide (20, 56);
un substrat marqué (26, 62) immobilisé dans la chambre d'interaction (22, 52) qui est configuré pour subir une interaction vérifiable avec des facteurs bactériens de bactéries, de sorte qu'il puisse être observé, par l'introduction dans la chambre d'interaction (22, 52) d'un milieu nutritif contenant un agent actif à analyser et les bactéries et par enregistrement du résultat de l'interaction vérifiable, si les bactéries sont résistantes à l'agent actif à analyser, les bactéries qui sont résistantes à l'agent actif à analyser pouvant former des facteurs bactériens correspondants,
dans lequel un volume d'espace vide des structures fluidiques n'est pas supérieur à 100 µl;
un détecteur (12, 66) qui est configuré pour enregistrer des informations qui sont une mesure de si l'interaction vérifiable a eu lieu ou non; et
une analyseur (14) qui est réalisé pour sortir, sur base des informatisons enregistrées, une indication concernant à résistante des bactéries à l'agent actif à analyser,
dans lequel le substrat (26, 62) est marqué par une substance qui est détachée du substrat (26, 62) par l'interaction avec les facteurs bactériens, et
dans lequel les structures fluidiques du module microfluidique présentent une chambre de détection (30, 54) qui est couplée en fluide à la chambre d'interaction (22, 52), dans lequel un dispositif de retenue (32, 60) est connecté en fluide entre la chambre d'interaction (22, 52) et la chambre de détection (30, 54), dans lequel le dispositif de rétention est configuré pour laisser passer les étiquettes (70) et/ou les fragments de substrat marqués qui se sont détachés du substrat marqué (26, 62) et pour retenir les bactéries (68).

2. Dispositif selon la revendication 1, dans lequel l'analyseur (14) est réalisé pour comparer les informations enregistrées avec au moins une valeur de seuil et pour sortir en fonction de la comparaison une indication de si les bactéries sont résistantes ou non à l'agent actif à analyser, ou dans lequel l'analyseur (14) est réalisé pour comparer les informations enregistrées avec plusieurs valeurs de seuil, et pour sortir en fonction de la comparaison une indication de si les bactéries sont résistantes, intermédiaires ou sensibles à l'agent actif à analyser.

3. Dispositif selon la revendication 1 ou 2, dans lequel la puce microfluidique (10, 50) présente une pluralité de films en matière plastique dont au moins un est structuré pour définir les structures fluidiques.

4. Dispositif selon l'une des revendications 1 à 3, dans lequel la puce microfluidique (10) présente par ailleurs une chambre d'enrichissement (40) qui est connectée en fluide entre l'entrée de fluide (20) et la chambre d'interaction (22).

5. Dispositif salon la revendication 4, qui présente un élément chauffant (28a) qui est configuré pour chauffer un contenu de la chambre d'enrichissement (40) à une température convenant pour un enrichissement des bactéries dans le milieu nutritif.

6. Dispositif selon l'une des revendications 1 à 5, qui présente un élément chauffant (28, 28 b) qui est configuré pour chauffer un contenu de la chambre d'interaction (22, 52) à une température convenant pour l'interaction vérifiable.

7. Dispositif selon l'une des revendications 1 à 6, dans lequel le substrat (26, 62) est marqué par des colorants fluorescents, des colorants absorbants ou des étiquettes marquées radioactives.

8. Dispositif selon l'une des revendications 1 à 7, dans lequel le substrat marqué (26, 62) est placé sur une paroi de la chambre d'interaction (22, 52) ou sur des structures fibreuses ou particulaires immobilisées dans la chambre d'interaction (22, 52).

9. Dispositif selon l'une des revendications 1 à 8, dans lequel la puce microfluidique (10, 50) présente plusieurs substrats marqués par différentes étiquettes (26, 62) qui sont immobilisés dans une ou plusieurs chambres à fluide de la puce microfluidique (10, 50).

10. Dispositif selon la revendication 9, dans lequel le substrat marqué (26, 62) est marqué par des fluorophores ou des colorants absorbants, et dans lequel le détecteur (12, 66) présente un photomultiplicateur ou un détecteur à un seul photon.

11. Procédé de détection de la résistance de bactéries à une agent actif à analyser à l'aide d'un dispositif selon l'une des revendications 1 à 10, aux caractéristiques suivantes consistant à:
introduire (100) le milieu nutritif qui continent l'agent actif à analyser et les bactéries dans la chambre d'interaction (22, 52) de la puce microfluidique (10, 50);
enregistrer (102) des informatisons qui sont une mesure de si l'interaction vérifiable a eu lieu ou non; et
sortir (104) une indication concertant la résistance des bactéries à l'agent actif à analyser sur base des informations enregistrées.

12. Procédé selon la revendication 11, qui présente le fait de comparer les informations enregistrées avec au moins une valeur de seuil et sortir, sur base de la comparaison, l'indication de si sont présentes des bactéries résistantes.

13. Procédé selon la revendication 12, qui présente le fait de comparer les informations enregistrées avec plusieurs valeurs de seuil et de sortir, sur la base de la comparaison, l'indication de si sont présentes des bactéries résistantes, intermédiaires ou sensibles à l'agent actif à analyser.

14. Procédé selon l'une des revendications 12 à 13, dans lequel les bactéries sont des bactéries connues et dans lequel le procédé est conçu pour effectuer un criblage d'agent actif.
